(19) Europäisches Patentamt
European Patent Office
Office européen des brevets

(11) EP 2 069 516 B1

(12) **EUROPEAN PATENT SPECIFICATION**

(45) Date of publication and mention of the grant of the patent:
**15.12.2010 Bulletin 2010/50**

(21) Application number: **07839163.8**

(22) Date of filing: **02.10.2007**

(51) Int Cl.:
***C12P 7/40*** $^{(2006.01)}$ ***C12P 7/62*** $^{(2006.01)}$
***C12P 41/00*** $^{(2006.01)}$

(86) International application number:
**PCT/US2007/021184**

(87) International publication number:
**WO 2008/042389 (10.04.2008 Gazette 2008/15)**

(54) **SPECIFIC HYDROLYSIS OF THE N-UNPROTECTED (R) -ESTER OF (3 ) -AMIN0-3-ARYLPR0PI0NIC ACID ESTERS**

SPEZIFISCHE HYDROLYSE DES UNGESCHÜTZTEN (R)-ESTERS VON (3)-AMINO-3-ARYLPROPIONSÄUREESTERN

HYDROLYSE SPÉCIFIQUE DE L'ESTER (R) NON PROTÉGÉ EN N DE L'ACIDE 3-AMINO-3-ARYLPROPIONIQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **03.10.2006 US 849162 P**

(43) Date of publication of application:
**17.06.2009 Bulletin 2009/25**

(73) Proprietor: **Dr. Reddy's Laboratories (EU) Limited**
**Beverley**
**East Yorkshire HU17 0LD (GB)**

(72) Inventors:
- **BYCROFT, Matthew**
  **Huntington, Cambs PE29 6JG (GB)**
- **CANTRILL, Alexander, A.**
  **Melbourne, Herts SG8 6UH (GB)**
- **KEENE, Philip, A.**
  **8810 Horgen (CH)**

(74) Representative: **Grünecker, Kinkeldey, Stockmair & Schwanhäusser**
**Anwaltssozietät**
**Leopoldstrasse 4**
**80802 München (DE)**

(56) References cited:
- **JUN OGAWA ET AL: "Microbial production of optically active [beta]-phenylalanine ethyl ester through stereoselective hydrolysis of racemic [beta]-phenylalanine ethyl ester" APPLIED MICROBIOLOGY AND BIOTECHNOLOGY, SPRINGER-VERLAG, BE, vol. 70, no. 6, 17 September 2005 (2005-09-17), pages 663-669, XP019421895 ISSN: 1432-0614**
- **LILJEBLAD ET AL: "Biocatalysis as a profound tool in the preparation of highly enantiopure beta-amino acids" TETRAHEDRON, ELSEVIER SCIENCE PUBLISHERS, AMSTERDAM, NL, vol. 62, no. 25, 19 June 2006 (2006-06-19), pages 5831-5854, XP005436637 ISSN: 0040-4020**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 069 516 B1

## Description

### Field of the Invention

The present invention relates to processes for the production of amino acid esters enriched in the (*S*)-enantiomer by bioresolution in the presence of an ester hydrolase, wherein the ester hydrolase is obtained from Aspergillus.

### Background of the Invention

Beta-amino acids and esters, such as 3-amino-3-arylpropionic acid esters have a wide variety of useful applications as synthetic intermediates, particularly for the manufacture of pharmaceutical agents. Such molecules are chiral, and it is preferable that they are accessible in the form of single enantiomers or at least in enantiomerically-enriched form.

Enantiomerically enriched forms of such molecules and the corresponding carboxylic acids can be prepared by way of chemical methodology. For example, United States Patent No. 6,673,926 B2 describes processes for making 3*S*-amino-3-arylpropionic acid (e.g. aryl = 3-pyridyl) and its derivatives using a classical resolution method that requires multiple steps including protection of the 3-amino group.

Biocatalytic methods have been described as more economically and ecologically advantageous alternatives for the preparation of entantiomerically enriched beta-amino acids and esters. For example, the use of a Lipase PS enzyme to give an (*S*)-acid has been described in United States Patent Publication 2004/0029236 A1, as well as in Faulconbridge, et al., Preparation of enantiomerically enriched aromatic β-amino acids via enzymatic resolution, Tetrahedron Letters 41 (2000) 2679-2681.

Other references describing biocatalytic methods for preparing enantiopure beta-amino acids and/or beta-amino esters include, for example, WO 01/16090A1, which includes a description of the molecules, but does not include a description of the method of synthesis of the enantiomerically enriched compounds. The following references describe the use of *Candida antartctica* Lipase to catalyse the enantioselective amide formation between the amino group of the racemic substrates and 2,2,2-trifluoroethyl esters in diisopropyl ether: M. Lui et al., Recent Advances in the stereoselective synthesis of β-amino acids, Tetrahedron 58 (2002) 7991-8035; S. Gedey et al., Preparation of highly enantiopure β-amino esters by Candida antarctica lipase A., Tetrahdron: Asymmetry 10 (1999) 2573-2581. Therefore, in the methods described by these references, only by use of an activated ester does acylation of the amino group preferentially occur at the (R)-center, leading to an enrichment of the (*S*)-unreacted substrate and formation of(R)-enriched amides. Similarly, V. Sanchez, et al., Candida antartctica lipase catalyzed resolution of ethyl (±)-3-aminohutyrate, Tetrahedron: Asymmetry, Vol. 8, No. 1, pp. 37-40, 1997, describes a similar route to the Gedey reference, but producing enantiopure 3-aminobutyric acid.

European Patent Application EP 1 057 894 A2, A3 describes an *Aspergillus flavus* esterase ATCC 11492 for use in preparation of *N*-benzyl-L-azetidine carboxylates to give the (*S*)-acid, but such enzymes have not been described for use in preparation of beta-amino acids or beta-amino esters.

The biocatalytic routes described in the above-identified prior art involving biocatalytic hydrolysis of a beta amino acid ester all give the (*S*')-acid as the product of hydrolysis. Should the corresponding (*S*)-ester then be required as a desired product, re-esterifrcation of the *(S)*-acid is required, normally by a chemical method. As a result, these biocatalytic routes require multiple process steps and thus are relatively lengthy and expensive.

Ogawa et al., Microbial production of optically active β-phenylalanine ethyl ester through stereoselective hydrolysis of racemic β-phenylalanine ethyl ester, Applied Microbiology and Biotechnology, Vol. 70, No. 6, pp. 663-669, 2006, describes the production of (S)- β-phenylalanine ethyl ester (BPAE) and (R)-β-phenylalanine (BPA) from racemic BPAE using cells of *Arthrobacter.*

### Summary of the Invention

In one aspect, the present invention is a process for the preparation of a 3-amino-3-arylpropionic acid ester that is enriched in the (*S*)-enantiomer to at least 80% ee, comprising: enantioselectively hydrolyzing an enantiomeric mixture of 3-amino-3-arylpropionic acid esters represented by formulae (*S*)-(**1**) and *R*-(**1**)

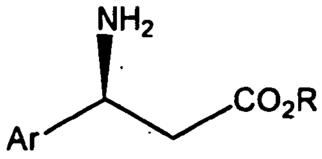

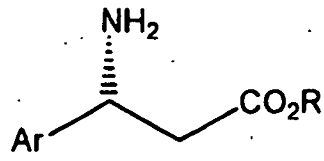

(*S*)-(**1**) (*R*)-(**1**)

wherein Ar is a $C_{4-30}$ unsubstituted or substituted aromatic group and R is a $C_{1-10}$ linear or branched alkyl group, in the presence of an ester hydrolase; and recovering the optically active ester (*S*)-(**1**) which is not hydrolyzed, and wherein the ester hydrolase is obtained from Aspergillus.

In a second aspect, the present invention is the use of an ester hydrolase obtained from *Aspergillus* for the preparation of a 3-amino-3-arylpropionic acid ester that is enriched in the (*S*)-enantiomer to at least 80% ee.

In a third aspect, the present invention is a process for the preparation of a 3-amino-3-arylpropionic acid that is enriched in the (*R*)-enantiomer to at least 80% enantiomeric excess (ee), comprising: enantioselectively hydrolyzing an enantiomeric mixture of 3-amino-3-arylpropionic acid esters represented by formulae (*S*)-(**1**) and (*R*)-(**1**)

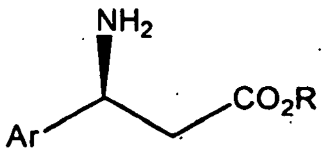

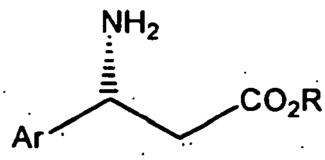

(*S*)-(**1**) (*R*)-(**1**)

wherein Ar is a $C_{4-30}$ unsubstituted or substituted aromatic group and R is a $C_{1-10}$ linear or branched alkyl group, in the presence of an ester hydrolase; and recovering the optically active acid (*R*)-(**2**), which is the product of the hydrolysis, wherein the ester hydrolase is obtained from Aspergillus.

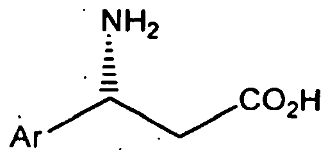

(*R*)-(**2**)

In a fourth aspect, the present invention is the use of an ester hydrolase obtained from *Aspergillus* for the preparation of a 3-amino-3-arylpropionic acid ester that is enriched in the (*R*)-enantiomer to at least 80% ee.

The present invention involves resolving the ester to leave behind the unreacted (*S*)-ester or to directly produce the *(R)*-acid.

Detailed Description of the Invention

Compounds prepared according to the present invention are (*S*)-enantiomer enriched amino acid esters represented by the formula (*S*)-(**1**):

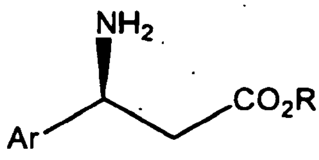

(*S*)-(**1**)

The amino acids are prepared from an enantiomeric mixture of 3-amino-3-arylpropionic acid esters represented by formulae (*S*)-(**1**) and (*R*)-(**1**):

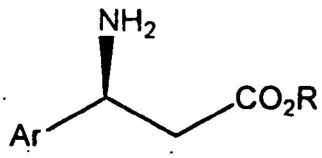

(*S*)-(**1**)

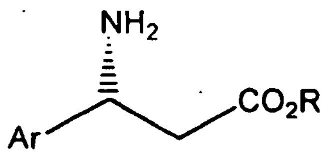

(*R*)-(**1**)

wherein Ar is a $C_{4-30}$ aromatic group and R is a $C_{1-10}$ alkyl group.

Suitable Ar groups include aryl, heteroaryl, substituted aryl or substituted heteroaryl. As used herein, unless otherwise noted, "aryl" shall refer to aromatic groups such as phenyl, napthyl, and the like. The aryl group may be unsubstituted or substituted with one or more substituents. Suitable substituents on the aryl group include but are not limited to those selected independently from the group consisting of halogen, alkyl, alkoxy, aralkyl, amino, amide, nitro, thio groups, carboxyl, hydroxy. As used herein, unless otherwise noted, "heteroaryl" shall denote any five or six membered monocyclic ring structure containing at least one heteroatom selected from O, N and S or a bicyclic ring system wherein the heteroaryl ring is fused to another aryl group. Examples of suitable heteroaryl groups include, but are not limited to, pyrrolyl, pyridyl, pyrazinyl, pyrimidinyl, pyrazolyl, pyridazinyl, furanyl, pyranyl, imidazolyl, thiophenyl, oxazolyl, isothiazolyl, isoxazolyl, furazanyl, benzothienyl, benzofuranyl, indolyl, isoindolyl, indolizinyl, indazolyl, purinyl, isoquinolyl, quinolyl, isothiazolyl, and the like. Preferably, Ar is phenyl or substituted phenyl, with phenyl being most preferred.

Suitable R groups include C1-10 linear or branched alkyl groups. As used herein, "alkyl" whether used alone or a part of a substituent group, shall include straight and branched chains containing 1 to 10 carbon atoms. For example, alkyl radicals include methyl, ethyl, n-propyl, isopropyl, n-butyl, isobutyl, sec-butyl, t-butyl, n-pentyl, n-hexyl and the like. Preferably, R is methyl, ethyl, propyl, isopropyl, butyl, isobutyl, or sec-butyl, with methyl or ethyl being most preferred.

In a particularly preferred embodiment, (*S*)-ethyl 3-amino-3-phenylpropionate is prepared from a mixture of (*S*)-ethyl 3-amino-3-phenylpropionate and (R)-ethyl 3-amino-3-phenylpropionate, as represented by the following formulae:

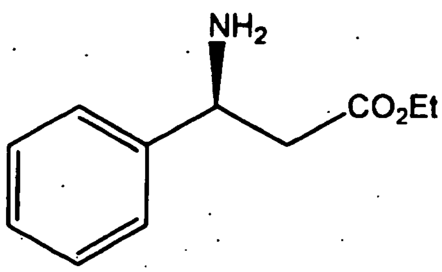

**(*S*)-ethyl 3-amino-3-phenylpropionate**

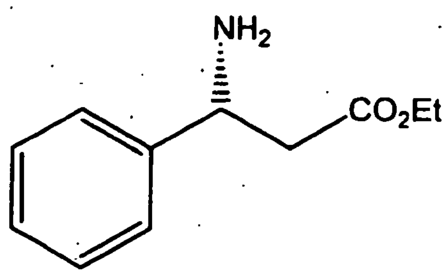

**(*R*)-ethyl 3-amino-3-phenylpropionate**

The 3-amino-3-aryl propionic acid esters of the present invention have an enantiomeric excess, or "ee" of the

(*S*)-enantiomer. "Enantiomeric Excess", or "ee" is a measure for how much of one enantiomer is present compared to the other, and is represented by the formula:

**% enantiomeric excess = (moles of major enantiomer - moles of other enantiomer / Total moles of both enantiomers) * 100**

Preferably, the 3-amino-3-arylpropionic acid esters of the present invention are enriched in the (*S*)-enantiomer to at least 80% ee, more preferably at least 90% ee, and even more preferably at least 95% ee.

The enzyme used in the present invention is an ester hydrolase obtained from the *Aspergillus* genus, and more preferably from the *Aspergillus flavus* species. Even more preferably, the ester hydrolase is an ester hydrolase from *Aspergillus flavus var. columnaris,* with *Aspergillus flavus var. columnaris* strain UKNCC 095253 and UKNCC 015958 being most preferred. The enzyme may be in aqueous solution, in a whole cell, in freeze dried cells, immobilized onto a solid support, retained within a membrane bioreactor, or a combination thereof.

An exemplary reaction scheme for the preparation of (*S*)-ethyl 3-amino-3-phenylpropionate is as follows:

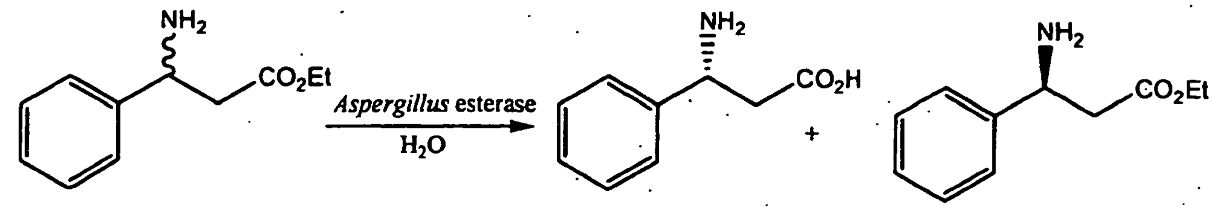


Desired product

The choice of reaction parameters is largely discretionary and appropriate conditions can be found using methods well known and routine in the art. As a guideline, the pH value range of the reaction should be between 4 and 10, preferably between 5 and 9 and more preferably between 6 and 8. If the pH is too high, there is a tendency for unselective chemical hydrolysis of the ester to occur. If the pH is too low, then the enzyme may degrade.

The reaction is preferably conducted at a temperature of greater than about 10 degrees centigrade, more preferably at least about 15 degrees C. The reaction temperature is preferably less than about 100 degrees C and more preferably less than about 40 degrees C.

The reactions of the present invention can be performed in any reaction vessel provided for the purpose, so long as the reaction vessel has appropriate pH and temperature control. For example, the reactions of the present invention can be performed in normal batch reactors, loop reactors or enzyme membrane reactors.

The reaction is typically performed under aqueous conditions such as in water and/or an aqueous buffer solution. If solubility of the reactants or reaction products is a concern, the reaction may also be performed in the presence of an organic co-solvent such as a hydrophobic organic solvent or a hydrophilic organic solvent in addition to the water or aqueous buffer solution. Such hydrophobic organic solvent may for example by an ether such as t-butyl methyl ether, an isopropyl ether or a hydrocarbon such as toluene, hexane, cyclohexane, heptane and isooctane, while a hydrophilic organic solvent may for example be an alcohol such as t-butanol, methanol, ethanol, isopropanol and n-butanol, an ether such as tetrahydrofuran, a sulfoxide such as dimethylsulfoxide, a ketone such as acetone or a nitrile such as acetonitrile. Each of these hydrophobic and hydrophilic solvents may be used as a sole co-solvent or in combination with each other, and a combination of a hydrophobic solvent and a hydrophilic solvent may also be contemplated.

The desired end product remains behind after the hydrolysis occurs and must be recovered. Preferably, the desired product is recovered by extraction, filtration or a combination whereof

Examples

The following examples illustrate certain embodiments of the invention and are intended to be illustrative only and not limiting.

Example 1 - Preparation of Substrate Solution (10.0 g/L 3-amino-3-phenyl-propionic acid ethyl ester in citric acid-sodium phosphate buffer):

The substrate solution was prepared by adding 0.2 g of ester (as prepared according to Tetrahedron, 2002, 58, 7449) to 20 ml of citric acid/sodium phosphate buffer (as prepared according to McIlvaine, J. Bio. Chem., 1921, 49, 183); the pH was adjusted to the required value with phosphoric acid.

Example 2 - Preparation of *Aspergillus* cells:

*Aspergillus flavus var. columnaris* was obtained from the United Kingdom National Culture Collection (UKNCC) as Strain Number 015958 (originally from the Collection of Filamentous Fungi and Plant Pathogenic Bacteria (CABI)) and stored as glycerol stocks at -70°C. This stock (250 μl) was used to inoculate 50 ml of sterile potato dextrose broth in a baffled 250 ml conical flask. Cultures were grown at 25°C, 250 rpm for 72 hours. This culture was then used to inoculate 2.0 L of sterile potato dextrose broth in a bench-top fermenter and the culture was grown at 25°C, 500 rpm, and 1.0 L air/min and with no pH control for 72 hours. The cells were harvested by filtration to yield 35.7 g wet cells which were then frozen at -20°C. The cells were then freeze dried to yield 7.25 g of dried cells.

Example 3 - Preparation of Biotransformation reaction mixture:

Substrate stock solution (20 ml) was incubated with 200 mg of freeze dried *Aspergillus flavus* harvested cells at 27°C (as a blank, in one reaction, cells were replaced with buffer).

Example 4 - Achiral analysis of biotransformation:

The reaction was stopped by transferring 100 μl of biotransformation mixture into 900 μl of HPLC solvent (20% acetonitrile + 10 mM potassium phosphate pH 3.0) for the achiral analysis.

Analytical:

Achiral HPLC Analysis was carried out using a C18 (BDS) column (150 x 4.6 mm, or equivalent) with HPLC solvent (20% acetonitrile + 10 mM potassium phosphate pH 3.0) at 2 ml/min, monitoring absorbance at 210 nm.

Example 5 - Chiral analysis of biotransformation:

For the chiral analysis 100 μl of biotransformation reaction was transferrer into 900 μl heptane, mixed, dried with anhydrous sodium sulphate and filtered, and the organic layer was analysed by HPLC.

Analytical:

Chiral Analysis can be performed to distinguish both isomers of the ester substrate. A Diacel Chiralcel OD-H column (250 x 4.6 mm, or equivalent) was used with 90% heptane/10% propan-2-ol as the mobile phase. A flow rate of 1 ml/min and detection at 210 nm was used.

Results:

For the biotransformation carried out at pH 7.0, a 17 hours reaction time gave 50% conversion (blank conversion 7%) and an isolated purity for the (*S*)-ester of >98.5 % ee.

## Claims

1. A process for the preparation of a 3-amino-3-arylpropionic acid ester that is enriched in the (*S*)-enantiomer to at least 80% enantiomeric excess (ee), comprising :

    enantioselectively hydrolyzing an enantiomeric mixture of 3-amino-3-arylpropionic acid esters represented by formulae (*S*)-(1) and (*R*)-(1)

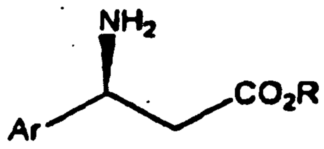 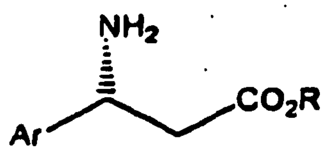

(*S*)-(1) (*R*)-(1)

wherein Ar is a $C_{4-30}$ unsubstituted or substituted aromatic group and R is a $C_{1-10}$ linear or branched alkyl group, in the presence of an ester hydrolase; and recovering the optically active ester (*S*)-(1) which is not hydrolyzed, and wherein the ester hydrolase is obtained from *Aspergillus,* preferably *Aspergillus flavus var. columnaris.*

2. The process according to claim 1, wherein R is methyl, ethyl, propyl, isopropyl, butyl, isobutyl or sec-butyl.

3. The process according to claim 1, wherein the enriched 3-amino-3-aryl propionic acid ester is (*S*)-3-amino-3-phenylpropionic acid ethyl ester.

4. The process according to any of claims 1 to 3 wherein the enantiomeric excess of the (S)-ester prepared is greater than 90%.

5. A process for the preparation of a 3-amino-3-arylpropionic acid that is enriched in the (*R*)-enantiomer to at least 80% enantiomeric excess (ee), comprising: enantioselectively hydrolysing an enantiomeric mixture of 3-amino-3-arylpropionic acid esters represented by formulae (*S*)-(1) and (*R*)-(1)

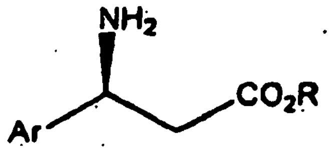 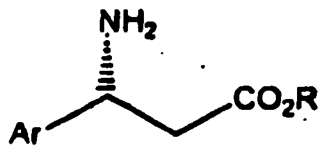

(*S*)-(1) (*R*)-(1)

wherein Ar is a $C_{4-30}$ unsubstituted or substituted aromatic group and R is a $C_{1-10}$ linear or branched alkyl group, in the presence of an ester hydrolase; and recovering the optically active acid (*R*)-(2) which is the product of the hydrolysis, wherein the ester hydrolase is obtained from *Aspergillus,* preferably *Aspergillus flavus var. columnaris.*

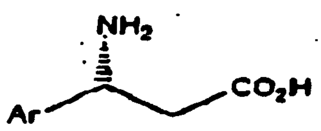

(*R*)-(2)

6. The process according to claim 5 wherein the enriched 3-amino-3-arylpropionic acid is (*R*)-3-amino-3-phenylpropionic acid.

7. The process according to any of claims 5 to 6 wherein the enantiomeric excess of the (*R*)-acid prepared is greater than 90%.

8. The process according to claim 1 or 5, wherein Ar is phenyl or substituted phenyl.

9. The process according to any one of claims 1 to 8 wherein the enzyme is in aqueous solution, whole cells, freeze

dried cells, immobilized onto a solid support, retained within a membrane bioreactor, or a combination thereof.

**10.** The process according to claim 9 wherein the enzyme of *Aspergillus flavus var. columnaris* is in isolated and purified form.

**11.** The process according to any one of claims 1 to 10, wherein the hydrolysis occurs at a pH of from 4 to 10, preferably from 6 to 8.

**12.** The process according to any one of claims 1 to 11, wherein the hydrolysis occurs at a temperature of from +5° C to +100° C, preferably from +15° C to +40° C.

**13.** Use of an ester hydrolase obtained from *Aspergillus,* preferably *Aspergillus raves var. columnaris* for the preparation of a 3-amino-3-arylpropio.nic acid ester that is enriched in the (*S*)-enantiomer to at least 80% ee.

**14.** Use of an ester hydrolase obtained from *Aspergilllus* preferably *Aspergillus flavus var. columnaris* for the preparation of a 3-amino-3-arylpropionic acid that is enriched in the (R)-enantiomer to at least 80% ee.

## Patentansprüche

**1.** Verfahren zur Herstellung eines 3-Amino-3-arylpropionsäureesters, welcher an dem (*S*)-Enantiomer bis wenigstens 80 % Enantiomeren-Überschuss (ee) angereichert ist, umfassend:

das enantioselektive Hydrolysieren eines Enantiomeren-Gemisches aus 3-Amino-3-arylpropionsäureestern, die durch die Formeln (*S*)-(1) und (*R*)-(1) wiedergegeben sind

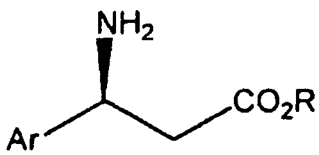

(*S*)-(1)

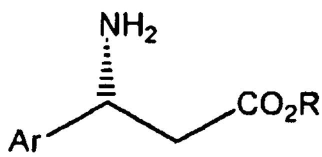

(*R*)-(1)

wobei Ar eine unsubstituierte oder substituierte aromatische $C_{4-30}$-Gruppe ist und R eine lineare oder verzweigte $C_{1-10}$-Alkylgruppe ist, in Gegenwart einer Esterhydrolase; und das Gewinnen des optisch aktiven Esters (*S*)-(1), welcher nicht hydrolysiert wird, und wobei die Esterhydrolase aus *Aspergillus,* vorzugsweise *Aspergillus flavus var. columnaris* gewonnen wird.

**2.** Verfahren nach Anspruch 1, wobei R Methyl, Ethyl, Propyl, Isopropyl, Butyl, Isobutyl oder sec-Butyl ist.

**3.** Verfahren nach Anspruch 1, wobei der angereicherte 3-Amino-3-arylpropionsäureester (*S*)-3-Amino-3-phenylpropionsäure-ethylester ist.

**4.** Verfahren nach einem der Ansprüche 1 bis 3, wobei der Enantiomeren-Überschuss des hergestellten (S)-Esters mehr als 90 % beträgt.

**5.** Verfahren zur Herstellung einer 3-Amino-3-arylpropionsäure, welche an dem (*R*)-Enantiomer bis wenigstens 80 % Enantiomeren-Überschuss (ee) angereichert ist, umfassend: das enantioselektive Hydrolysieren eines Enantiomeren-Gemisches aus 3-Amino-3-arylpropionsäureestern, die durch die Formeln (*S*)-(1) und (*R*)-(1) wiedergegeben sind

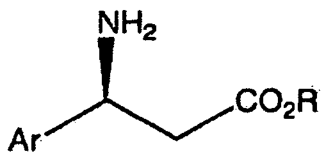

(*S*)-(1)

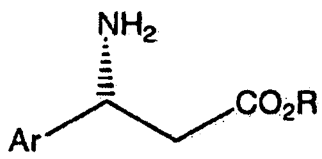

(*R*)-(1)

wobei Ar eine unsubstituierte oder substituierte aromatische $C_{4-30}$-Gruppe ist und R eine lineare oder verzweigte $C_{1-10}$-Alkylgruppe ist, in Gegenwart einer Esterhydrolase; und das Gewinnen der optisch aktiven Säure (*R*)-(2), welche das Produkt der Hydrolyse ist, wobei die Esterhydrolase aus *Aspergillus,* vorzugsweise *Aspergillus flavus var. columnaris* gewonnen wird.

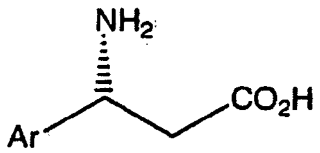

(*R*)-(2)

**6.** Verfahren nach Anspruch 5, wobei die angereicherte 3-Amino-3-arylpropionsäure (*R*)-3-Amino-3-phenylpropionsäure ist.

**7.** Verfahren nach einem der Ansprüche 5 bis 6, wobei der Enantiomeren-Überschuss der hergestellten (*R*)-Säure mehr als 90 % beträgt.

**8.** Verfahren nach Anspruch 1 oder 5, wobei Ar Phenyl oder substituiertes Phenyl ist.

**9.** Verfahren nach einem der Ansprüche 1 bis 8, wobei das Enzym in wässriger Lösung, ganzen Zellen, gefriergetrockneten Zellen, auf einem festen Träger immobilisiert, innerhalb eines Membranbioreaktors zurückgehalten, oder eine Kombination davon ist.

**10.** Verfahren nach Anspruch 9, wobei das Enzym von *Aspergillus flavus var. columnaris* in isolierter und gereinigter Form vorliegt.

**11.** Verfahren nach einem der Ansprüche 1 bis 10, wobei die Hydrolyse bei einem pH von 4 bis 10, vorzugsweise von 6 bis 8 stattfindet.

**12.** Verfahren nach einem der Ansprüche 1 bis 11, wobei die Hydrolyse bei einer Temperatur von +5 °C bis +100 °C, vorzugsweise von +15 °C bis +40 °C stattfindet.

**13.** Verwendung einer aus *Aspergillus,* vorzugsweise *Aspergillus flavus var. columnaris* erhaltenen Esterhydrolase für die Herstellung eines 3-Amino-3-arylpropionsäureesters, welcher an dem (*S*)-Enantiomer bis wenigstens 80 % ee angereichert ist.

**14.** Verwendung einer aus *Aspergillus,* vorzugsweise *Aspergillus flavus var. columnaris* erhaltenen Esterhydrolase für die Herstellung einer 3-Amino-3-arylpropionsäure, welche an dem (*R*)-Enantiomer bis wenigstens 80 % ee angereichert ist.

## Revendications

**1.** Procédé pour la préparation d'un ester d'acide 3-amino-3-arylpropionique enrichi en l'énantiomère (S) jusqu'à un excès énantiomérique (ee) d'au moins 80 %, comprenant :

l'hydrolyse énantiosélective d'un mélange d'énantiomères d'esters d'acide 3-amino-3-arylpropionique représentés par les formules (*S*)-(1) et (*R*)-(1) :

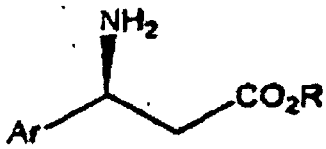

(*S*) – (1)

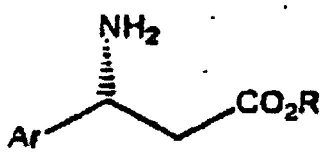

(*R*) – (1)

dans lesquelles Ar est un groupe aromatique substitué ou non substitué en $C_4$ à $C_{30}$ et R est un groupe alkyle linéaire ou ramifié en $C_1$ à $C_{10}$,
en présence d'une ester hydrolase ;
et la récupération de l'ester optiquement actif (*S*)-(1) qui n'est pas hydrolysé,
l'ester hydrolase étant obtenue à partir *d'Aspergillus,* de préférence *Aspergillus flavus var. columnaris.*

**2.** Procédé selon la revendication 1, dans lequel R est le radical méthyle, éthyle, propyle, isopropyle, butyle, isobutyle ou sec-butyle.

**3.** Procédé selon la revendication 1, dans lequel l'ester d'acide 3-amino-3-arylpropionique enrichi est l'ester éthylique d'acide (*S*)-3-amino-3-phénylpropionique.

**4.** Procédé selon l'une quelconque des revendications 1 à 3, dans lequel l'excès énantiomérique de l'ester (*S*) préparé est supérieur à 90 %.

**5.** Procédé pour la préparation d'un acide 3-amino-3-arylpropionique enrichi en l'énantiomère (R) jusqu'à un excès énantiomèrique (ee) d'au moins 80 %, comprenant :

l'hydrolyse énantiosélective d'un mélange d'énantiomères d'esters d'acide 3-amino-3-arylpropionique représentés par les formules (*S*)-(1) et (*R*)-(1) :

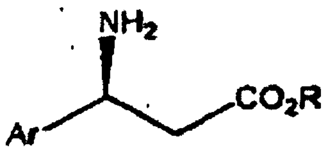

(*S*) – (1)

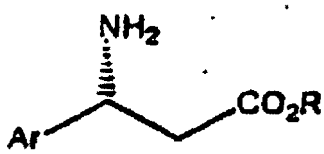

(*R*) – (1)

dans lesquelles Ar est un groupe aromatique substitué ou non substitué en $C_4$ à $C_{30}$ et R est un groupe alkyle linéaire ou ramifié en $C_1$ à $C_{10}$,
en présence d'une ester hydrolase ;
et la récupération de l'acide optiquement actif (*R*)-(2), qui est le produit de l'hydrolyse,
l'ester hydrolase étant obtenue à partir *d'Aspergillus,* de préférence *Aspergillus flavus var. columnaris* :

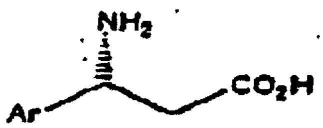

(*R*) – (2)

6. Procédé selon la revendication 5, dans lequel l'acide 3-amino-3-arylpropionique enrichi est l'acide (R)-3-amino-3-phénylpropionique.

7. Procédé selon l'une quelconque des revendications 5 et 6, dans lequel l'excès énantiomérique de l'acide (R) préparé est supérieur à 90 %.

8. Procédé selon la revendication 1 ou 5, dans lequel Ar est un radical phényle ou phényle substitué.

9. Procédé selon l'une quelconque des revendications 1 à 8, dans lequel l'enzyme est en solution aqueuse, cellules entières, cellules lyophilisées, immobilisée sur un support solide, retenue à l'intérieur d'un bioréacteur à membrane, ou une de leurs combinaisons.

10. Procédé selon la revendication 9, dans lequel l'enzyme d'*Aspergillus flavus var. columnaris* est sous une forme isolée et purifiée.

11. Procédé selon l'une quelconque des revendications 1 à 10, dans lequel l'hydrolyse a lieu à un pH de 4 à 10, de préférence de 6 à 8.

12. Procédé selon l'une quelconque des revendications 1 à 11, dans lequel l'hydrolyse a lieu à une température de +5°C à +100°C, de préférence de +15°C à +40°C.

13. Utilisation d'une ester hydrolase obtenue à partir *d'Aspergillus,* de préférence *Aspergillus flavus var. columnaris,* pour la préparation d'un ester d'acide 3-amino-3-arylpropionique enrichi en l'énantiomère (*S*) jusqu'à un ee d'au moins 80 %.

14. Utilisation d'une ester hydrolase obtenue à partir *d'Aspergillus,* de préférence *Aspergillus flavus var. columnaris,* pour la préparation d'un acide 3-amino-3-arylpropionique enrichi en l'énantiomère (R) jusqu'à un ee d'au moins 80 %.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6673926 B2
- US 20040029236 A1
- WO 0116090 A1
- EP 1057894 A2


### Non-patent literature cited in the description


- **Faulconbridge et al.** Preparation of enantiomerically enriched aromatic β-amino acids via enzymatic resolution. *Tetrahedron Letters,* 2000, vol. 41, 2679-2681
- **M. Lui et al.** Recent Advances in the stereoselective synthesis of β-amino acids. *Tetrahedron,* 2002, vol. 58, 7991-8035
- **S. Gedey et al.** Preparation of highly enantiopure β-amino esters by Candida antarctica lipase A. *Tetrahdron: Asymmetry,* 1999, vol. 10, 2573-2581
- **V. Sanchez et al.** Candida antartctica lipase catalyzed resolution of ethyl (±)-3-aminohutyrate. *Tetrahedron: Asymmetry,* 1997, vol. 8 (1), 37-40
- **Ogawa et al.** Microbial production of optically active β-phenylalanine ethyl ester through stereoselective hydrolysis of racemic β-phenylalanine ethyl ester. *Applied Microbiology and Biotechnology,* 2006, vol. 70 (6), 663-669
- *Tetrahedron,* 2002, vol. 58, 7449
- **McIlvaine.** *J. Bio. Chem.,* 1921, vol. 49, 183